# EUROPÄISCHE PATENTSCHRIFT

(11) **EP 0 880 059 B1**
(45) Veröffentlichungstag und Bekanntmachung des Hinweises auf die Patenterteilung: **04.10.2000**
(21) Anmeldenummer: 97108193.0
(22) Anmeldetag: 21.05.1997
(51) Int. Cl.: G03C 1/18, G03C 1/06

(54) **Photographisches Silberhalogenidmaterial und Verfahren zur Herstellung von Silberbildern**
Silver halide photographic material and process for forming silver images
Matériau photographique à l'halogénure d'argent et procédé de formation d'images argentiques

(43) Veröffentlichungstag der Anmeldung: 25.11.1998
(73) Patentinhaber: AGFA-GEVAERT N.V., 2640 Mortsel (BE)
(72) Erfinder: Rüger, Reinhold, Dr., 63322 Rödermark (DE); Varescon, Francois, Dr., 63263 Neu-Isenburg (DE)

(56) Entgegenhaltungen:
- DE-A- 3 337 985
- DE-A- 3 338 988
- GB-A- 1 207 006
- US-A- 3 615 634
- US-A- 4 546 074
- PATENT ABSTRACTS OF JAPAN vol. 16, no. 9 (P-1296), 10.Januar 1992 & JP 03 230155 A (FUJI PHOTO FILM CO. LTD.), 14.Oktober 1991,

## Beschreibung

Die Erfindung betrifft ein photographisches Silberhalogenidmaterial für die Herstellung von Silberbildern mit den Merkmalen des Oberbegriffs im Anspruch 1 und ein Verfahren zur Herstellung von Silberbildern.

Die Verwendung von Polymethin- oder Cyaninfarbstoffen zur spektralen Sensibilisierung von Silberhalogenidemulsionen ist seit langem bekannt. Zahlreiche Typen dieser Farbstoffe sind in einschlägigen Handbüchern, z.B. T.H. James "The Theory of the Photographie Process", 4^{th} edition, Seite 194 ff., Mac Millan Publishing Co. New York, 1977, beschrieben. Weitere Farbstoffe sind in US-A-3 615 634, GB-A- 1 207 006, DE-A-3 3 38 988, US-A-4 546 074, DE-A-3 337 985 und JP-A-3 230 155 beschrieben. Jedoch sind nicht alle Farbstoffe dieser Klasse gleichermaßen für die Anwendung in Silberhalogenidemulsionen geeignet.

An photographische Silberhalogenidmaterialien, beispielsweise für die Herstellung von schwarz-weißen Silberbildern für Anwendungen in der Druckvorstufe, werden eine Reihe von besonderen Anforderungen gestellt:
- Sie müssen eine möglichst hohe spektrale Empfindlichkeit haben.
- Sie müssen in kurzer Zeit verarbeitet werden können.
- Die Bilder dürfen auch bei kurzer Verarbeitungszeit keine störende Färbung durch Reste des Sensibilisierungsfarbstoffs aufweisen.

In den letzten Jahren ist es gebräuchlich geworden, den Verarbeitungszyklus für belichtete Silberhalogenidfilme, der üblicherweise aus einer Behandlung mit mindestens drei verschiedenen wäßrigen Bädern zur Entwicklung, Fixage und Wässerung besteht, durch Erhöhung der Entwicklerbadtemperatur und durch Verwendung konzentrierterer Entwickler- und Fixierbadlösungen zu verkürzen. Dabei werden jedoch die Sensibilisatorfarbstoffe häufig nicht mehr hinreichend vollständig aus der Schicht entfernt und es bleibt eine störenden Restfärbung zurück. Diese kann sich beispielsweise bei Schwarz-Weiß-Bildern als unzulässig hohe Minimaldichte gegenüber Licht bestimmter Wellenlänge oder bei Farbbildern als Verfälschung des Farbtons zeigen.

Es ist zwar in gewissen Grenzen möglich, die Sensibilisierungswirkung, d.h. die spektrale Empfindlichkeit zu steigern, indem man die Menge des Sensibilisatorfarbstoffs, bezogen auf die Menge des Silberhalogenids, erhöht. Jedoch steigt dann in der Regel auch die Menge des nach der Verarbeitung in der bildtragenden Schicht zurückbleibenden Farbstoffs und damit die Restfärbung an.

Es ist bekannt, daß die Entfärbbarkeit der photographischen Materialien bei der wäßrigen Verarbeitung durch wasserlöslichmachende Gruppen am Farbstoffmolekül gefördert wird. Solche Gruppen sind vor allem saure Gruppen wie die Sulfosäuregruppe oder die Carboxylgruppe.

In der EP-A1-04 27 892 werden gewisse dreikernige Merocyaninfarbstoffe als Sensibilisatoren für den roten Spektralbereich beschrieben, die eine gute Empfindlichkeit bei geringer Restfärbung ermöglichen sollen. Zumindest zwei der Substituenten an den Molekülen dieser Farbstoffe sollen spezielle wasserlöslich machende Gruppen in Form einer freien Säure, eines Salzes oder in latenter Form tragen. Beispiele solcher Gruppen sind die Carboxyl- und die Sulfosäuregruppe sowie Alkyl- und Arylgruppen, die mit einer dieser Säuregruppen substituiert sind.

Ein Silberhalogenidmaterial für Schnellverarbeitung zu ultrasteilem Kontrast mit geringer Restfärbung auf der Basis einer chloridreichen Emulsion mit einer Hydrazinverbindung und einer kontraststeigernden Aminoverbindung beschreibt die internationale Anmeldung WO 93/02389. Als Sensibilisator dient hier ein Benzimidazolcarbocyanin mit mindestens einer säuresubstituierten Alkylgruppe an einem der Imidazolstickstoffatome.

Die US-A-4,725,532 lehrt, daß der Pfefferkornschleier bei Hochkontrastmaterialien mit Hydrazinverbindungen und einem kationischen Cyanin-, Hemicyanin- oder Rhodacyaninfarbstoff verbessert wird, wenn diese zusätzlich Ascorbinsäure enthalten.

EP-A1-05 21 632 behandelt Trimethinfarbstoffe auf der Basis von Benzthiazol, die in der rotempfindlichen Schicht von Farbfilmen eine erhöhte Empfindlichkeit ergeben und eine geringe Neigung zu Farbverfälschungen durch Diffusion in eine andere Schicht haben sollen.

Insbesondere im Hinblick auf die Erzeugung von Silberbildern durch Belichtung von Silberhalogenidfilmen mit rotemittierenden Lichtquellen, vorzugsweise Lasern, und die anschließende Schnellverarbeitung besteht weiter Bedarf an rotempfindlichen Materialien mit hoher Empfindlichkeit und minimaler Restfärbung

Aufgabe der Erfindung ist es daher, ein photographisches Material mit hoher Empfindlichkeit im roten Wellenlängenbereich bereitzustellen, welches auch bei Schnellverarbeitung Bilder mit sehr geringer Restfärbung ergibt. Eine weitere Aufgabe ist es, ein Verfahren anzugeben, mit dem Silberbilder sehr geringer Restfärbung hergestellt werden können.

Diese Aufgaben werden gelöst durch ein photographisches Silberhalogenidmaterial nach Anspruch 1 und ein Verfahren nach Anspruch 7.

Es wurde nämlich überraschenderweise gefunden, daß photographische Silberhalogenidmaterialien, die in der Silberhalogenid-Emulsionsschicht einen Sensibilisatorfarbstoff der allgemeinen Formel (I) enthalten, 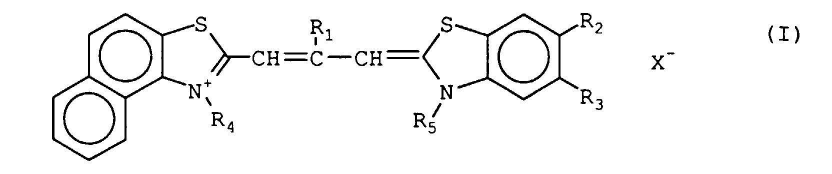 bei hoher Empfindlichkeit gegenüber Licht einer Wellenlänge zwischen 600 und 690 nm Bilder mit sehr geringer Restfärbung ergeben.

In der Formel (I) bedeuten R₁ eine Alkylgruppe mit 1 bis 6 Kohlenstoffatomen oder einen heterocyclischen Ring, R₂ Methoxy, R₃ Wasserstoff, Methyl oder Methoxy, R₄ und R₅ Alkylgruppen mit 1 bis 6 Kohlenstoffatomen, die mit Hydroxylgruppen substituiert sein können, und X⁻ ein Anion.

Dieses Ergebnis ist für den Fachmann überraschend, da die erfindungsgemäßen Farbstoffe in ihrem Molekül keine der genannten bekannten löslichmachenden Gruppen enthalten.

Die Gruppe R₁ kann beispielsweise Methyl, Ethyl, Propyl, Butyl, Isopropyl, Isobutyl, t-Butyl, gerades oder verzweigtes Pentyl oder Hexyl, Cyclopentyl, 1-, 2-, 3- oder 4-Piperidyl, 2-, 3- oder 4-Pyridyl, 2-, 3- oder 4-Morpholyl, 1-, 2- oder 3-Pyrrolidinyl, 2- oder 3-Tetrahydrothiophen-yl, 2- oder 3-Thiophen-yl, 2-, 3-, 4- oder 5-Thiazolinyl, 1-, 3- oder 4-Pyrazolyl, 1-, 2- oder 4-Imidazolyl sein.

Beispiele für R₄ und R₅ sind Methyl, Ethyl, Propyl, Butyl, Isopropyl, Isobutyl, t-Butyl, gerade oder verzweigtes Pentyl oder Hexyl, Hydroxymethyl, 1- oder 2-2-Hydroxyethyl, 2- oder 3-Hydroxypropyl.

Für die Ausführung der Erfindung sind zum Beispiel folgende Sensibilisatorfarbstoffe geeignet:

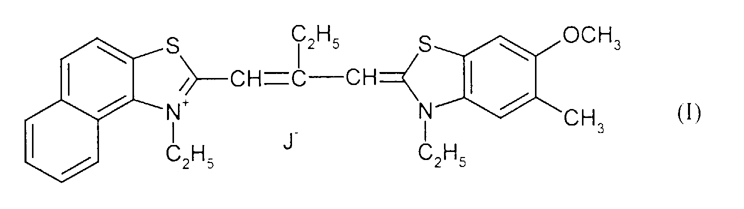

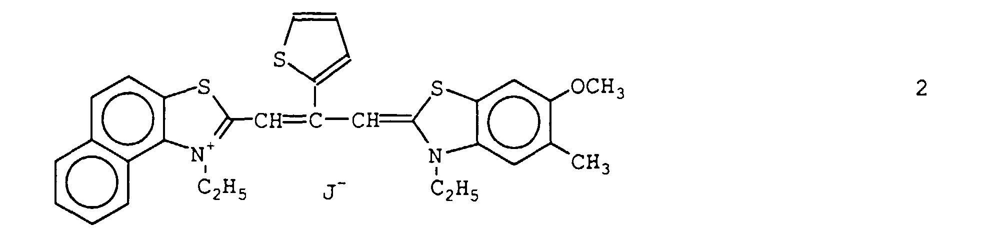

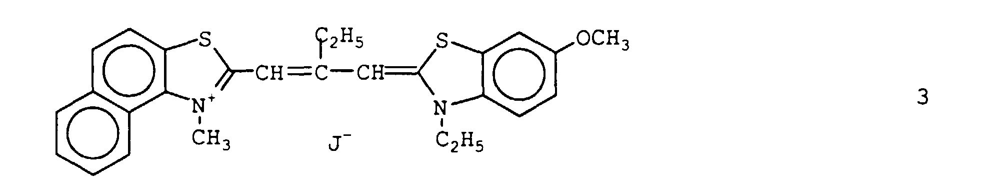

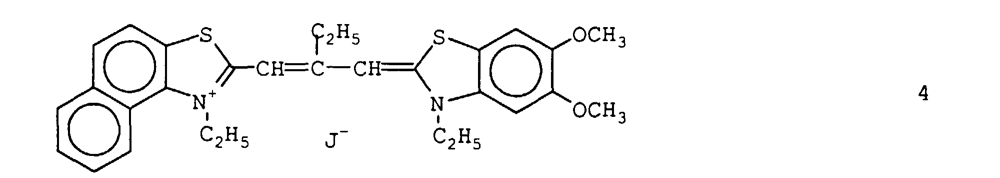

Die Menge des Sensibilisatorfarbstoffs kann in weiten Grenzen variiert werden. Ein bevorzugter Bereich liegt zwischen 10⁻⁶ und 10⁻² mol Farbstoff je mol Silberhalogenid.

Der Farbstoff kann auf bekannte Weise der Emulsion einverleibt werden, beispielsweise als wäßrige Lösung, als Lösung in nichtwäßrigen Lösungsmitteln wie Alkoholen oder Ketonen oder als feinteilige Suspension des Feststoffs in wäßriger Phase.

Die Emulsion der lichtempfindlichen Silberhalogenid-Emulsionsschicht ist vorzugsweise so sensibilisiert, daß sie negativ arbeitet, d. h. daß bei unmittelbarer Behandlung des bildmäßig belichteten Materials mit einem Negativentwickler, beispielsweise einem der im Beispiel 1 unten angegebenen Zusammensetzung, in den belichteten Bereichen Silberhalogenid zu Silber reduziert wird.

In einer bevorzugten Ausführungsform der Erfindung enthält das Aufzeichnungsmaterial eine Hydrazinverbindung. Solche Hydrazinverbindungen sind bekannt zur Steigerung des Kontrastes des Silberbildes. Die Hydrazinverbindung kann in an sich bekannter Weise entweder in eine oder mehrere Schichten des Aufzeichnungsmaterials inkorporiert werden. Dies können sowohl Schichten sein, welche das lichtempfindliche Silberhalogenid enthalten, als auch Schichten, die mit den erstgenannten in reaktiver Verbindung stehen, d. h. die so angeordnet sind, daß Stoffe von einer in die andere Schicht diffundieren können, wenn durch Reaktionen ein Konzentrationsgefälle aufrechterhalten wird.

Geeignete Hydrazinverbindungen sind beispielsweise beschrieben in Research Disclosure 235 010 (November 1983),
DE-27 25 743-A1, EP-00 32 456-B1, EP-01 26 000-A2,
EP-01 38 200-A2, EP-02 03 521-A2, EP-02 17 310-A2,
EP-02 53 665-A2, EP-03 24 391-A2, EP-03 24 426-A2,
EP-03 26 443-A2, EP-03 56 898-A2, EP-04 73 342-A1,
EP-05 01 546-A1, EP-04 81 565-A1, EP-05 98 315-A1,
EP-04 44 506-A.

Bevorzugte Hydrazinverbindungen sind durch die allgemeine Formel (H) beschrieben:

B - Phenyl - NHNH - L - G (H)

Hierin bedeuten B eine Ballastgruppe, G eine aktivierende Gruppe und L eine der Gruppen -CO- und -CO-CO-. "Phenyl" bedeutet einen Benzolring, an den B und die Hydrazingruppe gebunden sind, und zwar bevorzugt in Para-Stellung.

Bevorzugte Ballastgruppen sind jene, die nicht elektronenanziehend sind, beispielsweise gerade oder verzweigte Alkylgruppen, (z. B. Methyl, Ethyl, n-Propyl, Isopropyl, n-Butyl, Isobutyl, n-Hexyl, n-Octyl, t-Octyl, n-Decyl, n-Dodecyl und ähnliche Gruppen), auch Alkoxygruppen, die als Alkyl eine der oben genannten Alkylgruppen enthalten, sowie Acylaminogruppen, wie Acetylamino, Propanoylamino, Butanoylamino, Octanoylamino, Benzoylamino, Alkyl- und Arylsulfonamido-, Guanidyl- und ähnliche Gruppen.

Die genannten Gruppen können ihrerseits mit herkömmlichen photographischen Ballastgruppen substituiert sein, wie sie von inkorporierten diffusionsfesten Kupplern und anderen immobilisierten photographischen Zusätzen bekannt sind. Solche Ballastgruppen enthalten typischerweise mindestens 8 Kohlenstoffatome und können aus relativ reaktionsträgen aliphatischen oder aromatischen Gruppen ausgewählt werden, beispielsweise Alkyl, Alkoxy, Phenyl, Alkylphenyl, Phenoxy, Alkylphenoxy, Arylacyl, Arylamido, Alkylpyridinium-1-ylamido und ähnlichen Gruppen.

Die Alkyl- und Alkoxygruppen enthalten einschließlich etwaiger Ballastgruppen vorzugsweise 1 bis 20, die Acylaminogruppen vorzugsweise 2 bis 21 Kohlenstoffatome. Es können aber bis zu 30 oder mehr Kohlenstoffatome in diesen Gruppen enthalten sein. Besonders bevorzugt sind Methoxyphenyl, Tolyl, ballastiertes Butyramidophenyl, Butylsulfonamido und Toluylsulfonamido.

Zu den bevorzugten Hydrazinverbindungen gehören jene, deren Ballastgruppe noch eine adsorptionsfördernde Gruppe enthält. Solche Gruppen fördern die Adsorption des Moleküls an der Oberfläche der Silberhalogenidkristalle und sind an sich bekannt. Sie enthalten typischerweise wenigstens ein Schwefel- oder Stickstoffatom, das einen Silberkomplex bilden kann oder sonst eine Affinität zur Silberhalogenidoberfläche hat. Bevorzugte Beispiele sind Thioharnstoff-, Thiuronium-, heterozyklische Thioamid- und Triazolgruppen.

G ist vorzugsweise Wasserstoff, ggf. substituiertes Alkyl (z.B. Methyl, Hydroxymethyl, Monofluormethyl, Pyridinomethyl, Phenoxymethyl, Alkoxymethyl wie Methoxymethyl), ggf. substituiertes Aralkyl (z.B. Benzyl, o-Hydroxybenzyl) und ggf. substituiertes Aryl (z.B. Phenyl, 3,5-Dichlorphenyl, o-Methansulfonamidophenyl, 4-Methansulfonylmethyl, 2-Hydroxymethylphenyl), wobei Alkylgruppen mit elektronenanziehenden Substituenten, beispielsweise kationischen Gruppen mit quaternärem Stickstoffatom, wie Pyridinium und Imidazolium, besonders bevorzugt sind.

G kann auch weiter substituiert sein, z. B. mit Alkyl, Aralkyl, Alkenyl, Alkinyl, Alkoxy, Aryl, substituiertem Amino, Ureido, Urethan, Aryloxy, Sulfamoyl, Carbamoyl, Alkyl- oder Arylthio, Alkyl- oder Arylsulfonyl, Alkyl- oder Arylsulfinyl, Hydroxy, Halogen, Cyan, Sulfo, Aryloxycarbonyl, Acyl, Alkoxycarbonyl, Acyloxy, Carbamid, Sulfonamid, Carboxyl, Phosphamid, Diacylamino, Imid.

G kann auch so gewählt werden, daß das L-G-Teil des Moleküls unter Ringbildung abgetrennt wird, wie dies z.B. in EP-B-02 53 665 beschrieben ist.

Beispiele geeigneter Hydrazinverbindungen sind:

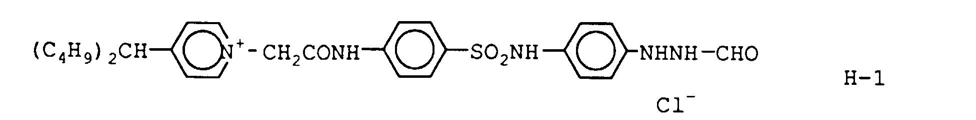

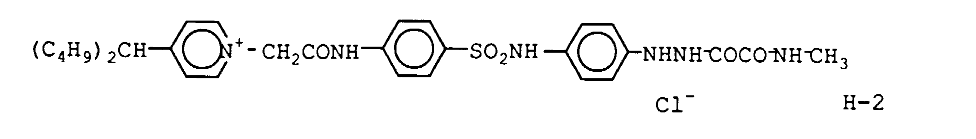

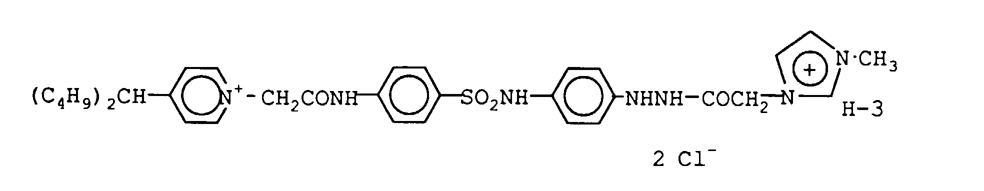

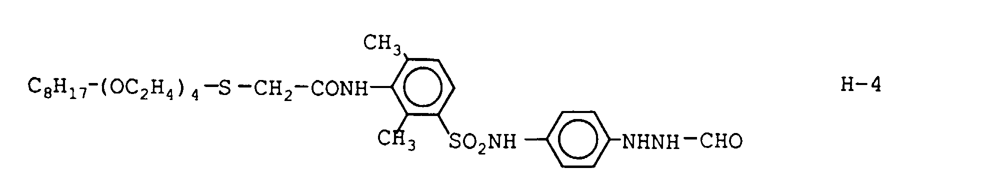

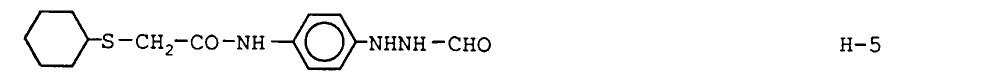

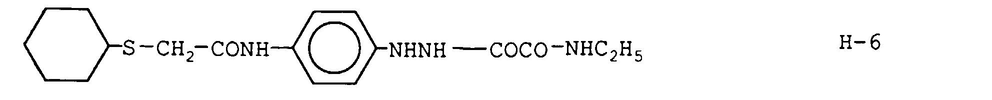

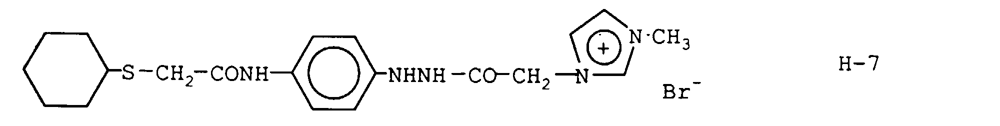

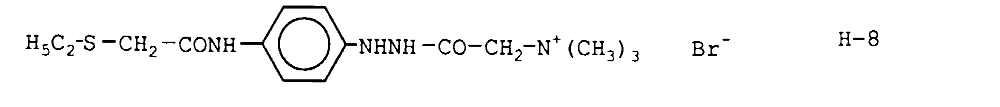

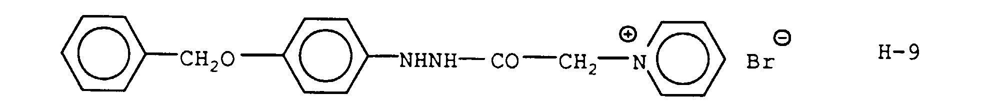

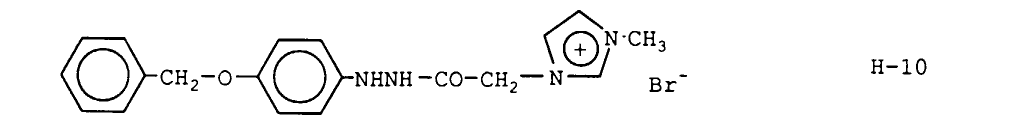

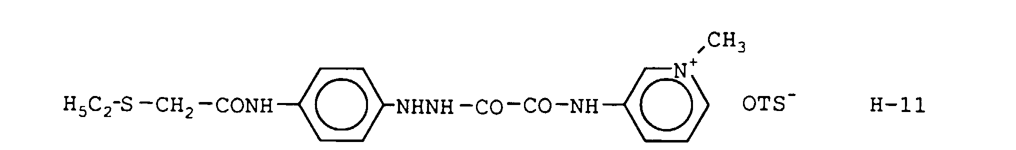

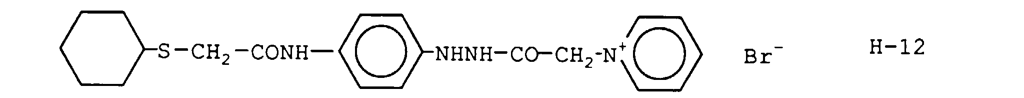

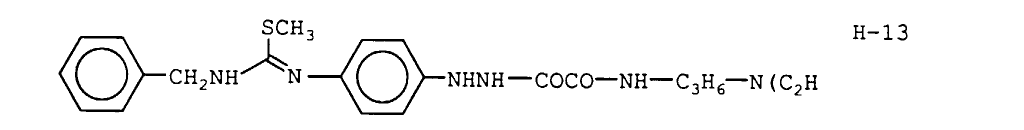

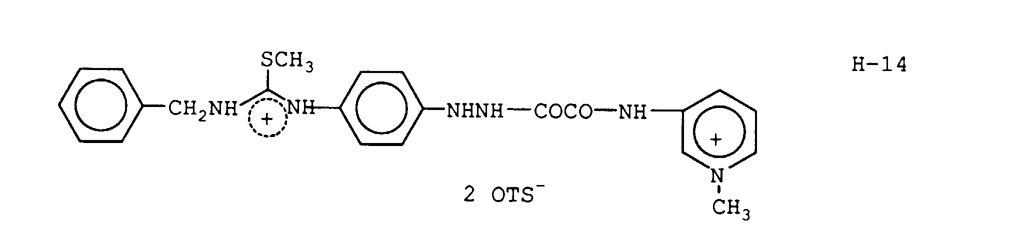

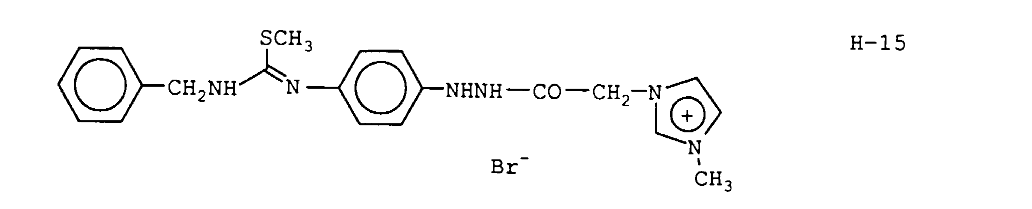

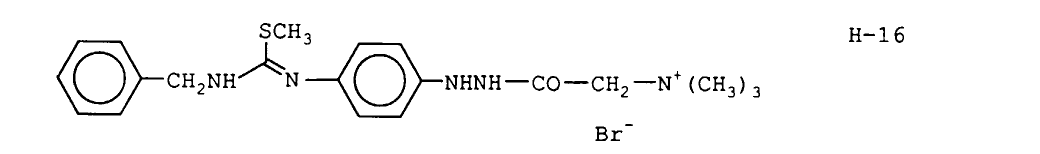

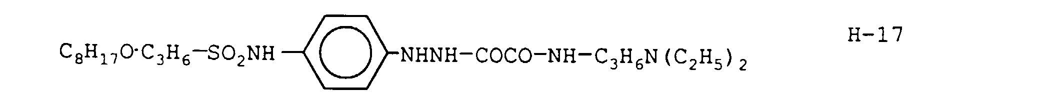

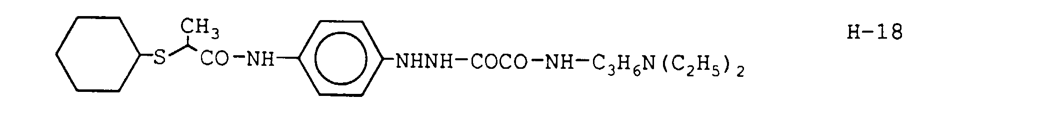

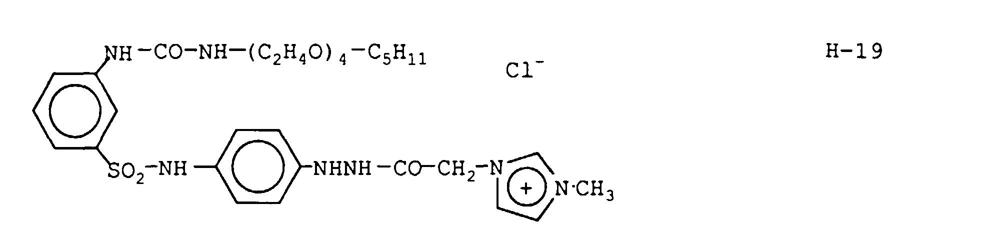

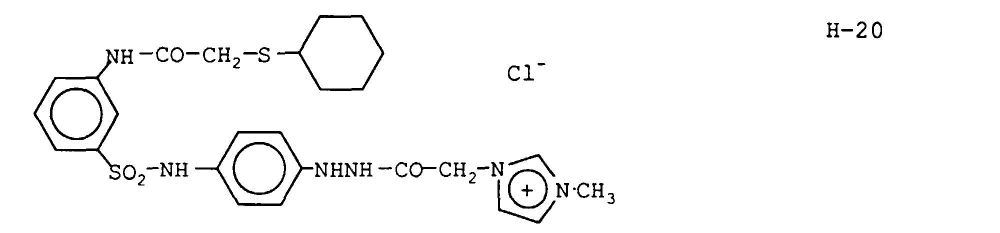

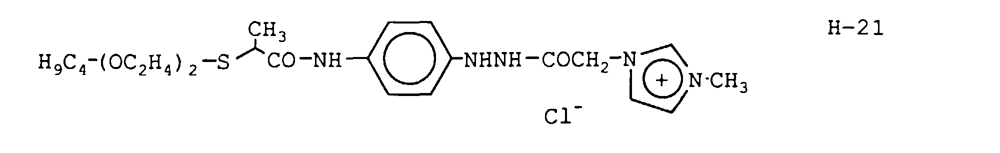

Die Menge der Hydrazinverbindung liegt bevorzugt zwischen 10⁻⁶ und 10⁻² mol je mol Silberhalogenid.

Weiter bevorzugt sind Aufzeichnungsmaterialien, die eine kontraststeigernde Aminoverbindung enthalten. Eine solche Aminoverbindung kann entweder allein oder zusätzlich zu der kontraststeigernden Hydrazinverbindung vorhanden sein.

Geeignete kontraststeigernde Aminoverbindungen sind beispielsweise bekannt aus US-A-4,914,003, EP-A-06 18 491 und EP-A-06 63 611.

Bevorzugt werden Aminoverbindungen, die in ihrem Molekül wenigstens eine sekundäre oder tertiäre Aminogruppe und darüber hinaus eine Gruppe mit einem quaternären Stickstoffatom, eine Polyoxyalkylenkette, eine Thioether- oder Thioketongruppe, eine Nitrilgruppe, eine Sulfonylharnstoff- oder -urethangruppe oder eine Guanidingruppe enthalten.

Beispiele geeigneter Aminoverbindungen sind:

(C₂H₅)₂N-C₃H₆―CN HCl A-1

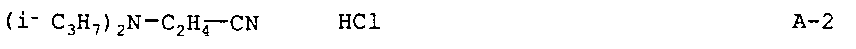

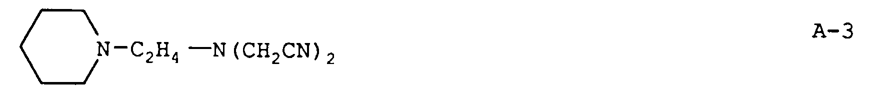

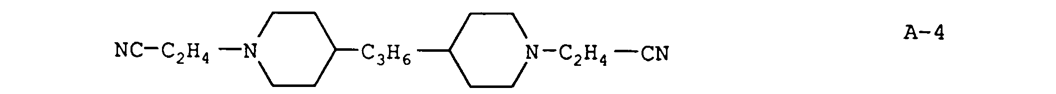

(C₂H₅)₂N-C₂H₄-O-C₂H₄-CN A-5

(C₂H₅)₂N-C₂H₄-S-C₂H₄-CN A-6

(C₂H₅)₂N-CH₂-CHOH-CH₂OH A-7

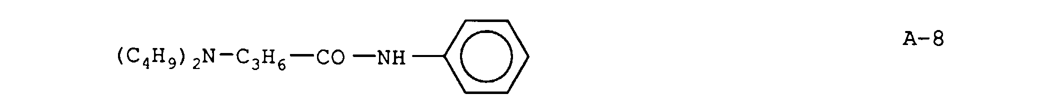

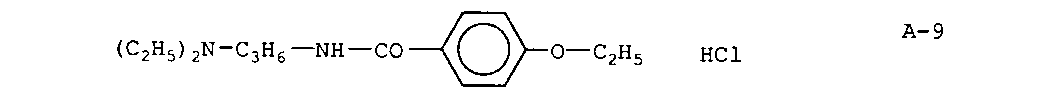

(C₃H₇)₂N-C₂H₄-(OC₂H₄)₁₄-N(C₃H₇)₂ A-10

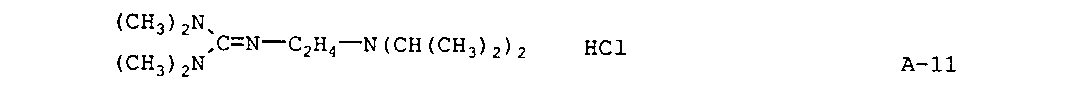

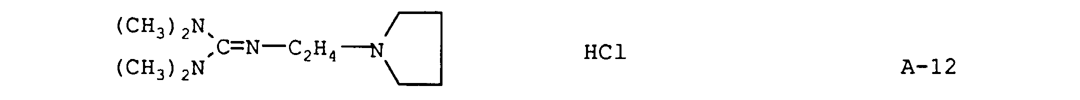

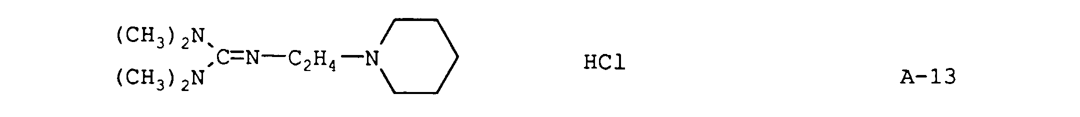

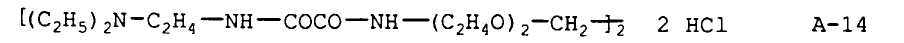

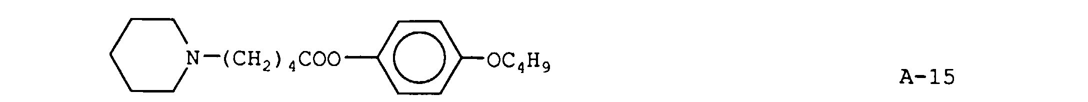

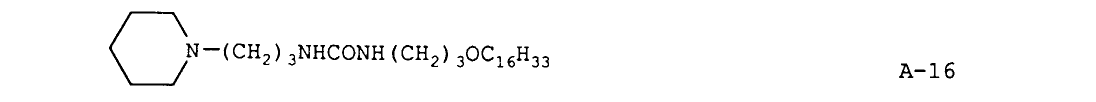

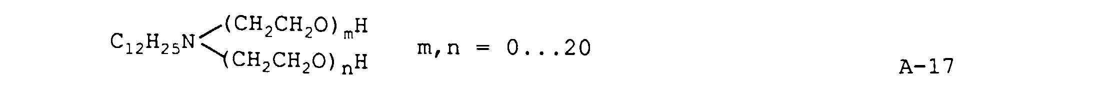

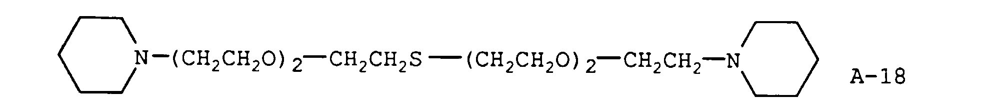

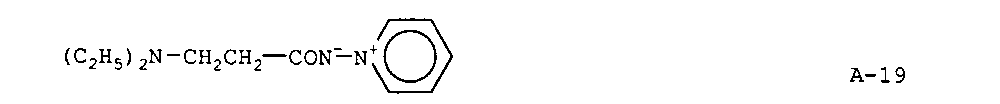

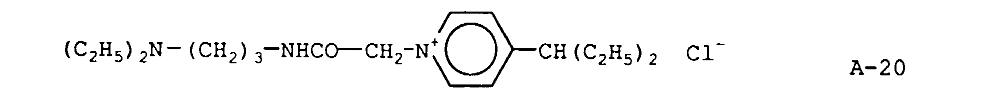

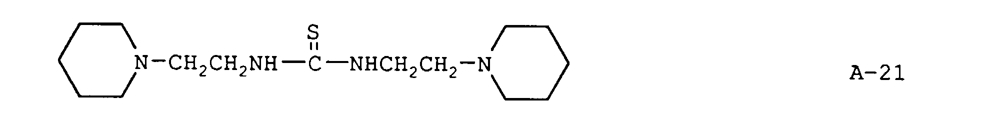

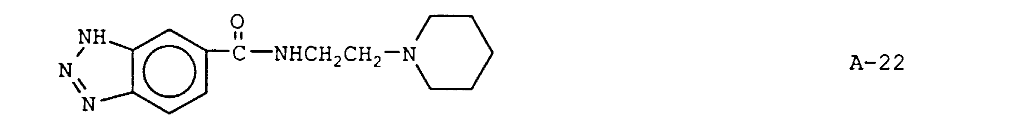

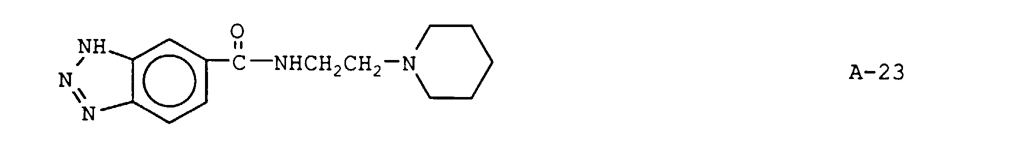

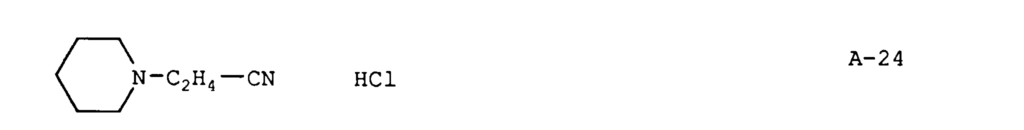

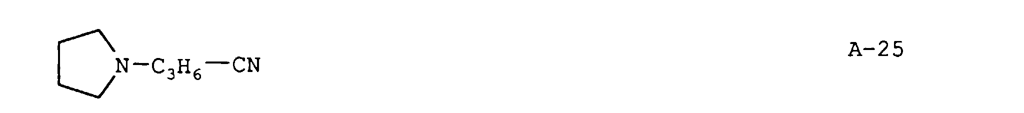

(C₄H₉)₂N-C₂H₄-CN HCl A-26

(C₄H₉)₂N-C₃H₆-N(CH₂CN)₂ A-27

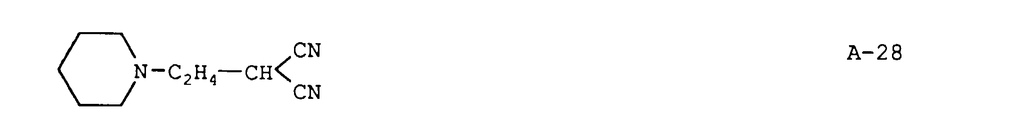

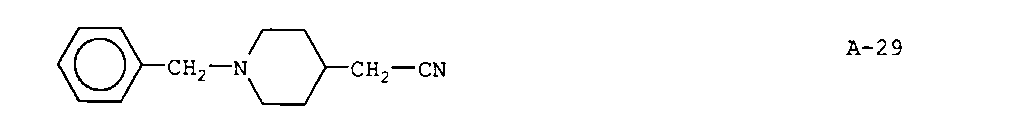

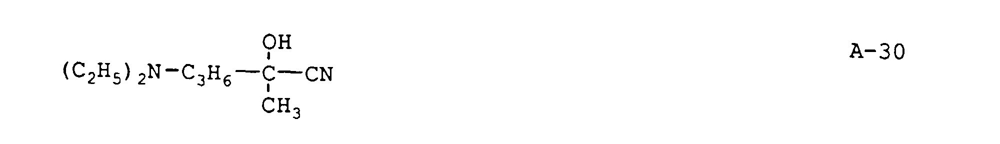

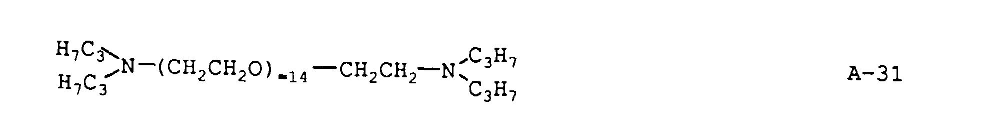

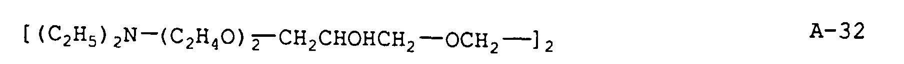

(C₂H₅)₂N-CH₂CHOHCH₂-O-CH₂CH₂-CH₂CHOHCH₂-N(C₂H₅)₂ A-33

[(C₂H₅)₂N-(CH₂CH₂O)₂-CH₂CHOHCH₂-OCH₂CH₂-]₂ A-34

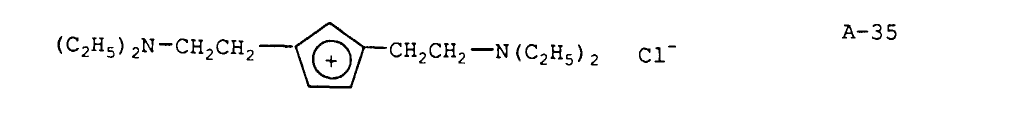

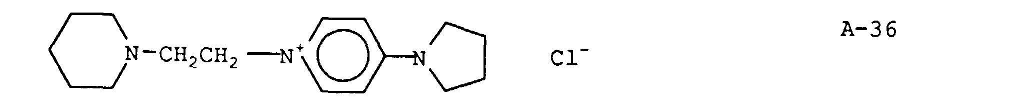

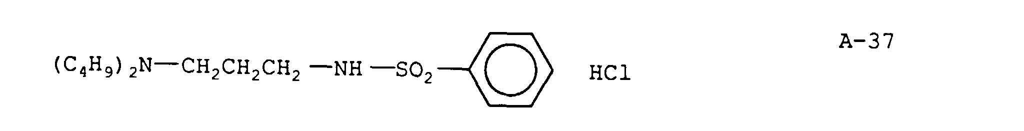

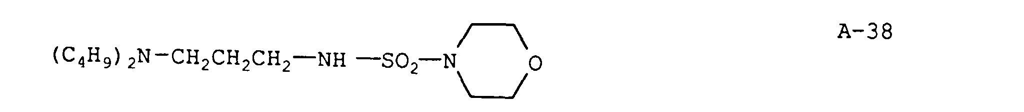

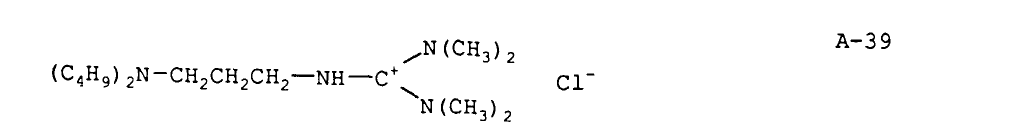

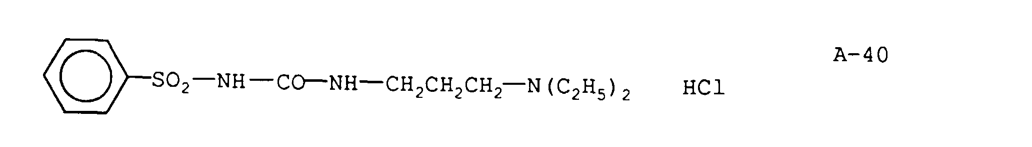

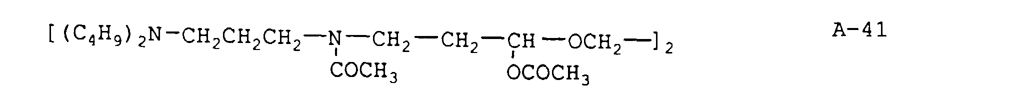

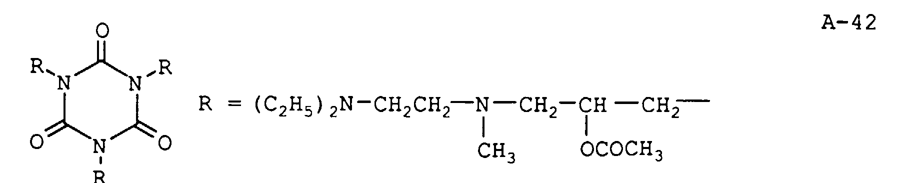

Die lichtempfindlichen Silberhalogenide der erfindungsgemäß verwendeten Aufzeichnungsmaterialien bestehen aus Silberchlorid, Silberbromid, Silberchlorobromid, Silberbromoiodid oder Silberchlorobromoiodid. Dabei sind Bromid und Bromoiodid bevorzugt. Sie können monodispers oder polydispers sein, eine einheitliche Zusammensetzung haben, aber auch Körner mit Kern-Schale-Aufbau aufweisen, sowie auch Gemische von Körnern verschiedener Zusammensetzung und Korngrößenverteilung sein. Sie werden unter Verwendung eines hydrophilen kolloidalen Bindemittels, bevorzugt Gelatine, hergestellt. Die Silberhalogenidkörner können sphärische, polyedrische oder tafelförmige Gestalt haben. Methoden zur Herstellung geeigneter lichtempfindlicher Silberhalogenidemulsionen sind dem Fachmann bekannt und beispielsweise in der Research Disclosure 365 044, Kapitel I bis IV (September 1994) zusammengefaßt.

Bevorzugt für die erfindungsgemäß verwendeten
Aufzeichnungsmaterialien werden Silberhalogenidemulsionen, die durch kontrollierten Doppelstrahleinlauf hergestellt werden und eine kubische Kornform haben. Vorteilhaft sind Emulsionen, bei denen mindestens 80 Gewichtsprozent des Silberhalogenids in kubischer Form vorliegen. Besonders bevorzugt sind monodisperse Emulsionen, d. h. solche, bei denen der Variationskoeffizient (Quotient aus Standardabweichung und Mittelwert) der Korngröße kleiner als 0,30 ist. Unter Korngröße wird die Kantenlänge eines mit dem wirklichen Korn volumengleichen Würfels verstanden.

Das Kornvolumen der Silberhalogenidkörner in den Emulsionen richtet sich nach der erforderlichen Empfindlichkeit und kann beispielsweise dem kubischer Körner von 0,1 bis 0,7 µm Kantenlänge entsprechen. Ein bevorzugter Bereich liegt zwischen 0,15 und 0,30 µm. Bei der Emulsionsherstellung können Edelmetallsalze, besonders Salze von Rhodium oder Iridium, zur Steuerung der photographischen Eigenschaften in den üblichen Mengen anwesend sein.

Die Emulsionen werden bevorzugt chemisch sensibilisiert. Geeignete Verfahren sind die Schwefel-, die Reduktions- und die Edelmetallsensibilisierung, die auch in Kombination angewendet werden können. Für letztere können beispielsweise Gold- oder Iridiumverbindungen benutzt werden. Die Sensibilisierung wird bevorzugt in Gegenwart von Salzen organischer Thiosulfonsäuren, wie der p-Toluolthiosulfonsäure, durchgeführt.

Die Emulsionen können auch übliche Antischleiermittel enthalten. Bevorzugt sind ggf. substituiertes Benztriazol, 5-Nitroindazol und 1-Phenyl-5-mercaptotetrazol. Diese Mittel können zu jedem Zeitpunkt bei der Emulsionsherstellung zugesetzt werden oder in einer Hilfsschicht des photographischen Materials enthalten sein. Zur Verbesserung der photographischen Eigenschaften kann der Emulsion vor oder nach der chemischen Reifung ein Jodid, vorzugsweise ein Alkalijodid, in einer Menge von etwa 0,5 bis 10 mmol je Mol Silber zugesetzt werden.

Die Emulsionen können auch bekannte Polymerdispersionen enthalten, durch die beispielsweise die Dimensionsstabilität des photographischen Materials verbessert wird. Es handelt sich dabei in der Regel um Latices hydrophober Polymere in wäßriger Matrix. Beispiele für geeignete Polymerdispersionen sind in der Research Disclosure 176 043, Kapitel IX B (Dezember 1978) genannt. Bevorzugt werden Polymere und Copolymere von Estern der Acryl- und der Methacrylsäure, besonders bevorzugt von C₁- bis C₆-Estern, z. B. Polyethylacrylat. Die Teilchengröße dieser Polymerlatices liegt bevorzugt zwischen 20 und 100 nm.

Die lichtempfindlichen Schichten der photographischen Materialien können durch Zusatz eines Härtungsmittels gehärtet sein. Härtungsmittel sind beispielsweise in der Research Disclosure 365 044, Kapitel II B (September 1994) genannt. Das Härtemittel kann der Emulsion zugesetzt oder über eine Hilfsschicht beispielsweise eine äußere Schutzschicht, eingebracht werden. Geeignete Härtemittel sind beispielsweise Aldehyde, wie Formaldehyd oder Glutaraldehyd, Vinylsulfone, s-Triazine, Aziridine, Carbodiimide, Carbamoylpyridiniumverbindungen, mono- und bifunktionelle Carbamoylimidazoliumverbindungen. Ein bevorzugtes Härtungsmittel ist Hydroxydichlorotriazin.

Das photographische Material kann weitere Zusätze, die für die Erzeugung bestimmter Eigenschaften bekannt und üblich sind, enthalten. Solche Mittel sind zum Beispiel in der Research Disclosure 365 044 (September 1994) in den Kapiteln VI (Aufheller), IX A (Beschichtungshilfsmittel), IX B (Weichmacher und Gleitmittel) und IX D (Mattierungsmittel) aufgeführt. Auch kann das Material, wenn es zur Erzeugung von Farbbildern bestimmt ist, Farbkuppler und andere Zusätze, die dort in den Kapiteln XII und XIII beschrieben sind, enthalten.

Der Gelatinegehalt der Emulsionen liegt im allgemeinen zwischen 30 und 150 g je mol Silber; bevorzugt wird der Bereich zwischen 40 und 100 g je mol Silber.

Zur Erfindung gehört auch ein Verfahren für die Herstellung von Silberbildern, welches dadurch gekennzeichnet ist, daß man ein vorstehend beschriebenes photographisches Material bildmäßig belichtet und danach verarbeitet, indem man es in einer wäßrigen Entwicklerlösung entwickelt, in üblicher Weise fixiert, wässert und trocknet.

Dieses Verfahren wird bevorzugt als Schnellverfahren mit einer Entwicklungszeit von höchstens 30 s und entsprechend angepaßter Entwicklertemperatur ausgeführt.

Die erfindungsgemäß verwendeten Entwicklerlösungen enthalten bevorzugt eine Dihydroxybenzol-Entwicklersubstanz, beispielsweise Hydrochinon, Brenzkatechin, Methylhydrochinon oder Chlorhydrochinon, und ein Oxydationsschutzmittel, bevorzugt ein Alkalisulfit in einer Konzentration von mehr als 0,3 mol je Liter. Besonders bevorzugt werden Lösungen mit pH-Werten von 9 bis höchstens 11. Solche Entwicklerlösungen sind auch im Gebrauch gut haltbar und ergeben weitgehend schleierfreie Bilder. Ebenfalls verwendbar sind Entwicklerlösungen mit einer Entwicklersubstanz vom Ascorbinsäuretyp, beispielsweise L-Ascorbinsäure, D-Ascorbinsäure, L-Erythroascorbinsäure, 6-Desoxy-L-ascorbinsäure, Imino-L-erythroascorbinsäure oder Zuckerderivate dieser Säuren. Geeignet sind auch Entwicklerlösungen, die sowohl Entwicklersubstanzen vom Dihydroxybenzol-Typ als auch solche vom Ascorbinsäuretyp enthalten.

Bevorzugt enthalten die Entwicklerlösungen bekannte superadditiv wirkende Hilfsentwicklersubstanzen, beispielsweise N-Methyl-p-aminophenol oder 1-Phenylpyrazolidinon-3 oder Derivate dieser Verbindungen.

Ebenfalls bevorzugt sind Entwickler, die Stabilisatoren aus den Gruppen der Benztriazole und Mercaptotetrazole enthalten. Solche Stabilisatoren sind beispielsweise 1-Phenyl-5-mercaptotetrazol, 1-(4-Hydroxyphenyl)-5-mercaptotetrazol, 1-(1-Naphthyl)-5-mercaptotetrazol, 1-Cyclohexyl-5-mercaptotetrazol, 1-(4-Chlorphenyl)-5-mercaptotetrazol, 1-(3-Capramidophenyl)-5-mercaptotetrazol, Benztriazol, 5-Chlorbenztriazol, 5-Brombenztriazol, 5-Methylbenztriazol, 5-Nitrobenztriazol, 5-Benzoylaminobenztriazol, 1-Hydroxymethylbenztriazol, 6-Cyanobenztriazol.

Die Erfindung kann zur Erzeugung Silberbildern, vorzugsweise von schwarz-weißen Negativbildern mit ultrasteilem Kontrast, insbesondere bei der Reproduktion in der Druckvorstufe für den Schwarz-Weiß- und Mehrfarbdruck, angewendet werden.

In den folgenden Ausführungsbeispielen sind die Mengen der Emulsionsbestandteile auf jeweils 1 mol Silber bezogen, soweit nichts anderes angegeben ist. Als Vergleichsverbindungen dienen die bekannten Farbstoffe V-1 bis V-8:

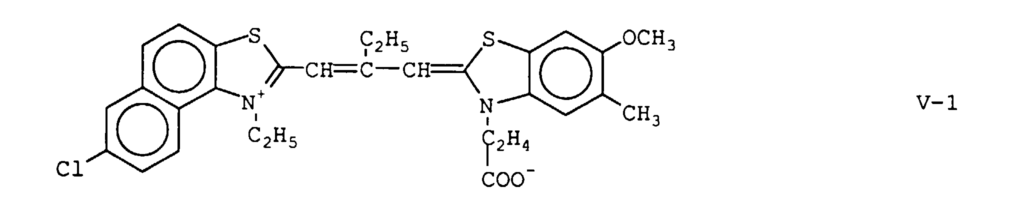

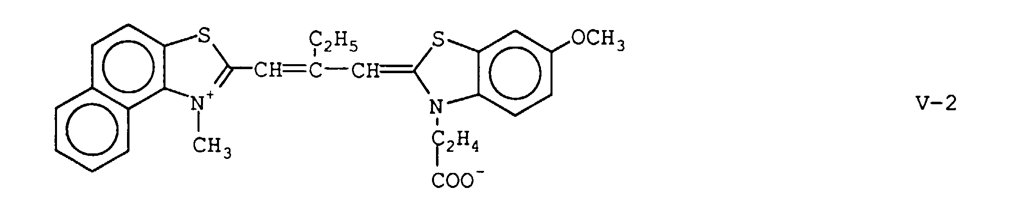

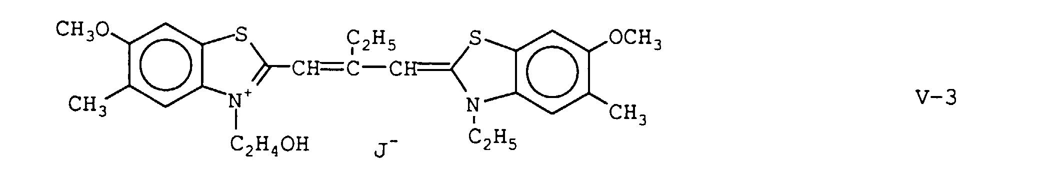

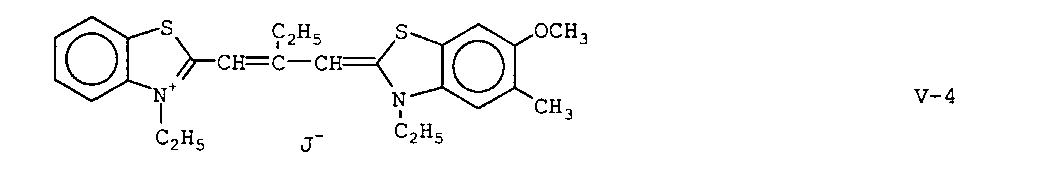

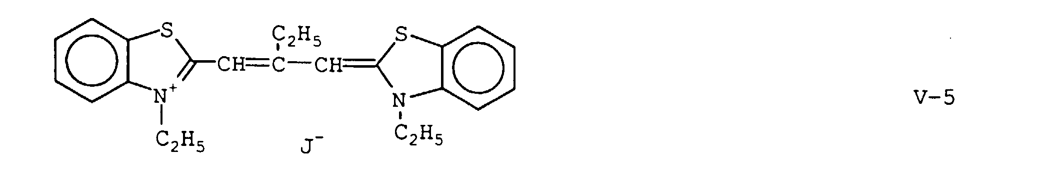

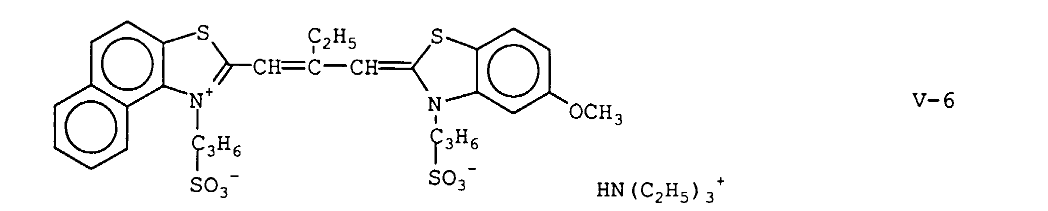

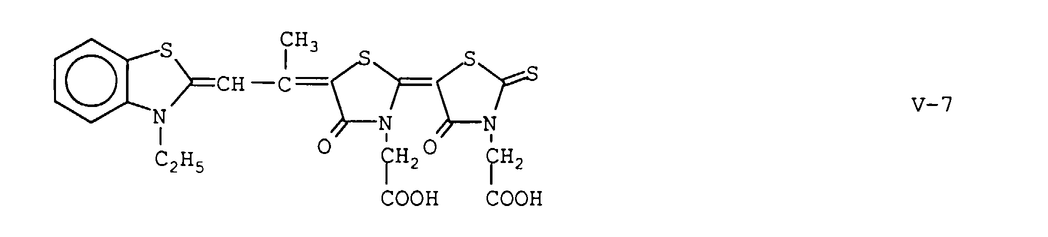

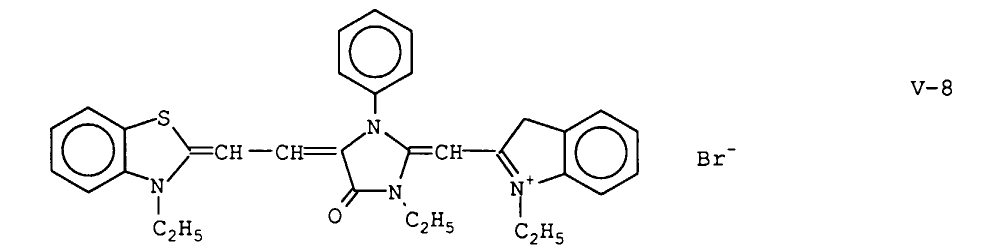

### Beispiel 1

Durch pAg-geregelte Zweistrahlfällung in einer Gelatinelösung wurde eine Silberbromoiodidemulsion mit kubischen Körnern der Kantenlänge 0,25 µm und einem Iodidanteil von 2 Molprozent hergestellt. Die Emulsion wurde mittels des Flockverfahrens von den löslichen Salzen befreit und das Koagulat mit weiterer Gelatine redispergiert. Die so erhaltene Emulsion wurde unter Zusatz von 0,2 millimol Thiosulfat und 0,03 millimol Tetrachlorogoldsäure chemisch gereift. Danach wurden Teilen dieser Emulsion noch 5 millimol Kaliumiodid, der in Tabelle 1 angegebene Sensibilisatorfarbstoff, 300 mg 7-Hydroxy-1-methyl-1.3.4-triazaindolizin, 100 mg 5-Nitroindazol, 4 g Hydrochinon, 15 g Polyethylacrylat als wäßriger Latex und ein Netzmittel zugefügt. Die so erhaltenen gießfertigen Emulsionen wurden gleichzeitig mit einer Schutzschicht-Überzugslösung auf einen Polyethylenterephthalat-Schichtträger aufgetragen. Die Schutzschicht enthielt je Quadratmeter 1 g Gelatine, 0,3 millimol Formaldehyd und 0,5 g kolloidale Kieselsäure sowie eine Polyethylendispersion und ein Mattierungsmittel. Die so erzeugten Aufzeichnungsmaterialien enthielten 4,5 g Silber je Quadratmeter.

Proben dieser Materialien wurden mit einem Blitzlicht (2∗10⁻³ s) durch ein Rotfilter und einen Dichteverlaufskeil belichtet, in einer Rollenentwicklungsmaschine mit einem Entwickler der unten angegebenen Zusammensetzung 30 s bei 35 °C entwickelt, mit einem handelsüblichen Fixierbad behandelt, 30 s gewässert und getrocknet. An den fertig verarbeiteten Proben wurde die Empfindlichkeit S als Kehrwert der für die Dichte 1,0 benötigten Belichtung, bezogen auf den Wert 100 für die Probe 1 gemessen sowie die Restfärbung visuell beurteilt. Die Ergebnisse sind in Tabelle 1 aufgeführt.

Zusammensetzung des Entwicklers (alle Bestandteile in g)

| | |
|---|---|
| Wasser | 500 |
| Natriumbisulfit | 50 |
| Kaliumhydroxid | 27 |
| Ethylendiamintetraessigsäure, Trinatriumsalz | 3,7 |
| Hydrochinon | 25 |
| Kaliumbromid | 4 |
| Benzotriazol | 0,3 |
| Phenylmercaptotetrazol | 0,05 |
| 4-Hydroxymethyl-4-methyl-1-phenylpyrazolidinon | 1 |
| Borsäure | 3 |
| Natriumhydroxid | 24 |
| Diethylenglykol | 40 |

Mit Wasser auf 1 l auffüllen, pH auf 10,5 bei 22 °C einstellen.

**Tabelle 1**

| Probe Nr. | Sensibilisator | | S | Restfärbung |
|---|---|---|---|---|
| | Stoff | Menge (mg) | | |
| 1 | V8 | 70 | 100 | blau |
| 2 | V7 | 150 | 400 | schwach rötlich |
| 3 | V6 | 70 | 160 | rot-violett |
| 4 | V5 | 80 | 350 | rot |
| 5 | V4 | 80 | 500 | schwach rot |
| 6 | V3 | 80 | 500 | schwach rot |
| 7 | V2 | 80 | 1000 | stark rot |
| 8 | V2 | 40 | 500 | stark rot |
| 9 | V1 | 80 | 500 | rot |
| 10 | 1 | 40 | 1100 | keine |
| 11 | 1 | 80 | 1500 | keine |
| 12 | 1 | 130 | 1700 | leicht rot |
| 13 | 2 | 40 | 500 | keine |
| 14 | 3 | 40 | 700 | keine |
| 15 | 3 | 80 | 1200 | leicht rötlich |
| 16 | 3 | 130 | 1300 | leicht rot |

### Beispiel 2

Eine Silberbromoiodidemulsion mit kubischen Körnern der Kantenlänge 0,17 µm und einem Iodidanteil von 2 Molprozent wurde durch pAg-geregelte Zweistrahlfällung in einer Gelatinelösung hergestellt. Die Emulsion wurde wie im Beispiel 1 geflockt, gewaschen und redispergiert und dann mit 0,01 millimol Hexachloroplatinsäure, 0,4 millimol Thiosulfat und 0,1 millimol Tetrachlorogoldsäure chemisch gereift. Danach wurden 5 millimol Kaliumiodid, 300 mg 7-Hydroxy-1-methyl-1.3.4-triazaindolizin, 200 mg 5-Nitroindazol, 0,15 millimol 1-Pyridiniumacetyl-2-(4-Benzyloxyphenyl)-hydrazinbromid (Verbindung H-9), 0,6 g N-(3-Dibutylaminopropyl)-benzolsulfonamidhydrochlorid (Verbindung A-37), 1,25 g Polyethylenoxid (Molmasse 4000), sowie jeweils Teilen der Emulsion die in Tabelle 2 angegebenen Sensibilisatorfarbstoffe zugesetzt.

Die so erhaltenen gießfertigen Emulsionen wurden auf einen auf der Rückseite mit Antistatik- und Anticurlbeschichtung versehenen Polyethylenterephthalat-Schichtträger gleichzeitig mit einer Beschichtungslösung für eine Schutzschicht mit 0,9 g/m² Gelatine aufgetragen. Das Silber-Flächengewicht betrug 3,5 g je Quadratmeter.

Proben der so erhaltenen Materialien wurden wie in Beispiel 1 beschrieben belichtet und zu Silberbildern verarbeitet. Neben Empfindlichkeit und Restfärbung wie dort wurden auch minimale und maximale Dichte Dmin und Dmax sowie die mittlere Gradation G zwischen den Dichtewerten 0,4 und 3,5 ermittelt.

Die in Tabelle 2 aufgeführten Ergebnisse zeigen, daß die erfindungsgemäßen Farbstoffe auch in Hochkontrastfilmen bei Schnellverarbeitung keine Restfärbung verursachen und die übrigen photographischen Eigenschaften nicht beeinträchtigen.

**Tabelle 2**

| Probe | Sensibilisator | | Dmin | Dmax | S | G | Restfärbung |
|---|---|---|---|---|---|---|---|
| | Stoff | Menge (mg) | | | | | |
| 1 | V7 | 170 | 0,053 | 6,2 | 100 | 20 | schwach rosa |
| 2 | V6 | 100 | 0,056 | 5,5 | 45 | 15 | schwach blau |
| 3 | V6 | 50 | 0,075 | 5,9 | 76 | 18 | stark blau |
| 4 | 1 | 100 | 0,052 | 6,1 | 120 | 16 | keine |
| 5 | 1 | 50 | 0,048 | 6,0 | 61 | 17 | keine |

### Beispiel 3

Proben einiger Materialien aus Beispiel 2 wurden auf einem Fotosatzbelichter mit Helium-Neon-Laser (Linotronic 300, Firma Hell) mit Rastertönen der Rasterweite 60 Linien je cm und mit Schriften belichtet. Die Proben wurden wie in Beispiel 2 verarbeitet, jedoch mit 25 s Entwicklungszeit. Die Empfindlichkeit wurde anhand der für einen 50%-Punkt erforderlichen Laserenergieeinstellung ermittelt. Punktqualität und Restfärbung wurden visuell beurteilt. Die Ergebnisse sind in Tabelle 3 angegeben.

**Tabelle 3**

| Probe | Empfindlichkeit | Punktqualität | Restfärbung |
|---|---|---|---|
| 1 | 100 | sehr gut | schwach rosa |
| 3 | 55 | sehr gut | blau |
| 4 | 200 | sehr gut | keine |
| 6 | 50 | ausreichend | schwach rosa |

Die zum Vergleich aufgenommene Probe 6 enthält keine Hydrazinverbindung und ist im übrigen gleich der Probe 1.

## Patentansprüche

1. Photographisches Silberhalogenidmaterial umfassend mindestens eine lichtempfindliche Silberhalogenid-Emulsionsschicht auf einem Schichtträger, wobei in dieser Emulsionsschicht wenigstens ein Sensibilisatorfarbstoff vorhanden ist, der das Silberhalogenid für Licht im Spektralbereich von 600 bis 690 nm sensibilisiert, dadurch gekennzeichnet, daß der Sensibilisatorfarbstoff unter die allgemeine Formel (I) fällt, 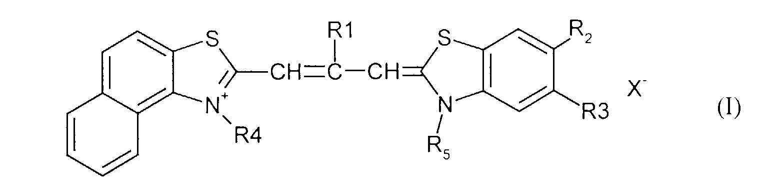 worin R₁ eine Alkylgruppe mit 1 bis 6 Kohlenstoffatomen oder einen heterocyclischen Ring, R₂ Methoxy, R₃ Wasserstoff, Methyl oder Methoxy, R₄ und R₅ Alkylgruppen mit 1 bis 6 Kohlenstoffatomen, die mit Hydroxylgruppen substituiert sein können, und X⁻ ein Anion bedeuten.

2. Material nach Anspruch 1, dadurch gekennzeichnet, daß die Silberhalogenid-Emulsionsschicht eine negativ arbeitende Schicht ist.

3. Material nach Anspruch 1 oder 2, dadurch gekennzeichnet, daß es in der Silberhalogenid-Emulsionsschicht oder in einer mit dieser in reaktiver Beziehung stehenden Schicht eine Hydrazinverbindung enthält.

4. Material nach einem der Ansprüche 1 bis 3, dadurch gekennzeichnet, daß es in der Silberhalogenid-Emulsionsschicht oder in einer mit dieser in reaktiver Beziehung stehenden Schicht eine kontraststeigernde Aminoverbindung enthält.

5. Material nach einem der Ansprüche 1 bis 4,,
dadurch gekennzeichnet, daß
der Sensibilisatorfarbstoff zur folgenden Gruppe gehört:

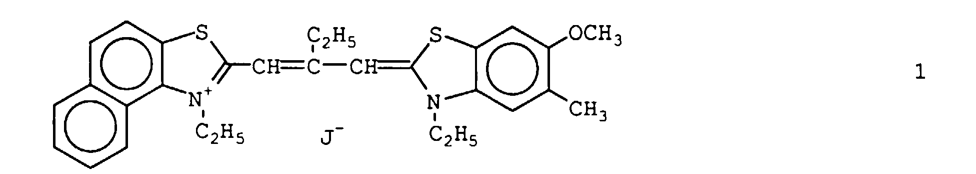

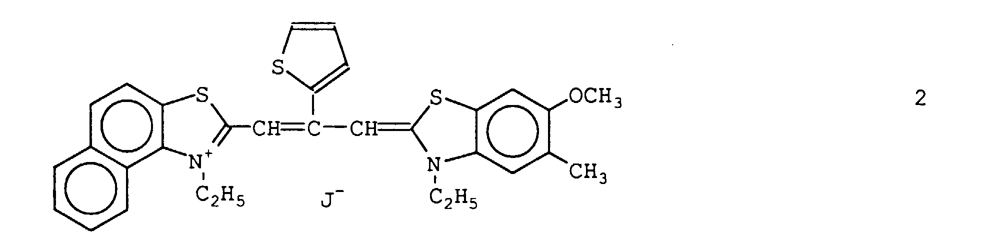

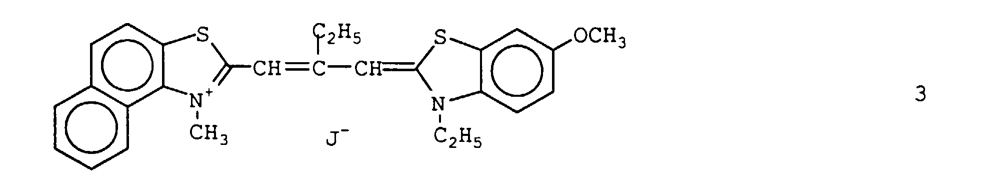

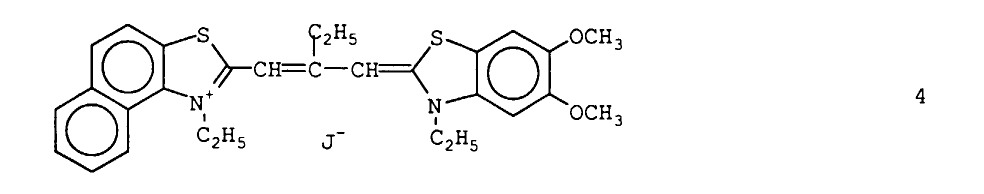

6. Material nach einem der Ansprüche 1 bis 4,
dadurch gekennzeichnet, daß
die Silberhalogenidemulsion eine Silberbromid- oder eine Silberbromoiodidemulsion ist.

7. Verfahren zur Herstellung eines Silberbildes,
dadurch gekennzeichnet, daß
man ein photographisches Aufzeichnungsmaterial nach einem der vorstehenden Ansprüche belichtet und verarbeitet.

8. Verfahren nach Anspruch 7,
dadurch gekennzeichnet, daß
die Entwicklungszeit höchstens 30 s beträgt.

## Claims

1. Photographic silver-halide material comprising at least one light-sensitive silver-halide emulsion layer on a layer support, where this emulsion layer contains at least one sensitizer dye which sensitizes the silver halide to light in the spectral region from 600 to 690 nm, characterized in that the sensitizer dye falls under the general formula (I) 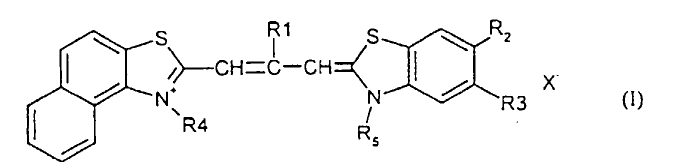 in which R₁ is an alkyl group having 1 to 6 carbon atoms or a heterocyclic ring, R₂ is methoxy, R₃ is hydrogen, methyl or methoxy, R₄ and R₅ are alkyl groups having 1 to 6 carbon atoms, which may be substituted by hydroxyl groups, and X⁻ is an anion.

2. Material according to Claim 1, characterized in that the silver-halide emulsion layer is a negative-working layer.

3. Material according to Claim 1 or 2, characterized in that it contains a hydrazine compound in the silver-halide emulsion layer or in a layer which is reactively connected to this layer.

4. Material according to one of Claims 1 to 3, characterized in that it contains a contrast-increasing amino compound in the silver-halide emulsion layer or in a layer which is reactively connected to this layer.

5. Material according to one of Claims 1 to 4, characterized in that the sensitizer dye belongs to the following group:

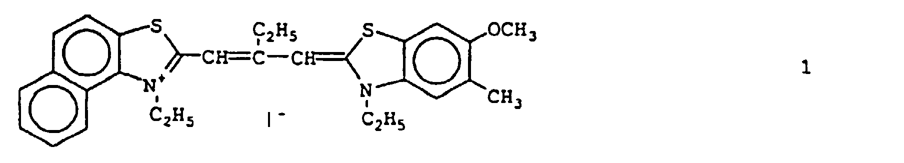

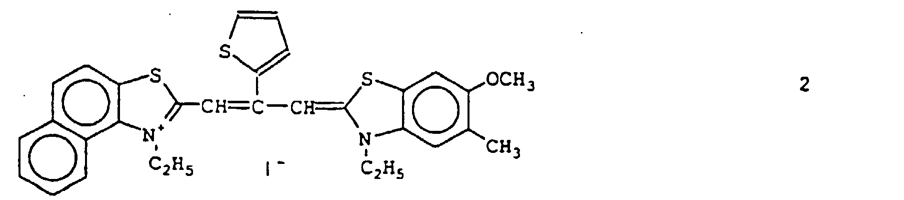

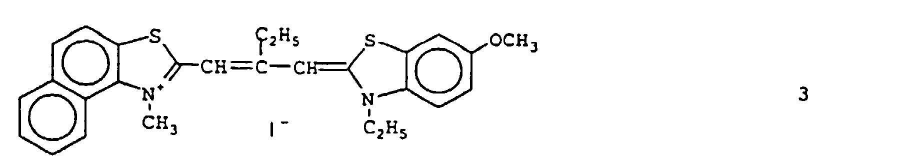

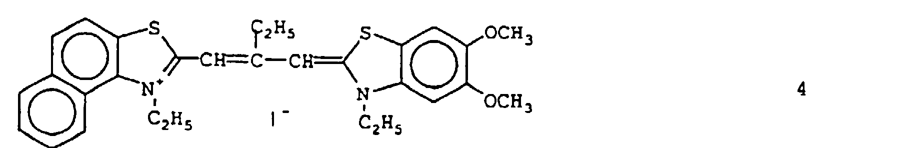

6. Material according to one of claims 1 to 4, characterized in that the silver-halide emulsion is a silver bromide or a silver bromoiodide emulsion.

7. Process for the production of a silver image, characterized in that a photographic recording material is exposed and processed according to one of the preceding claims.

8. Process according to claim 7, characterized in that the development time is at most 30 seconds.

## Revendications

1. Matériau photographique à l'halogénure d'argent comprenant au moins une couche d'émulsion photosensible à l'halogénure d'argent sur un support de couches, dans lequel au moins un colorant de sensibilisation est présent dans cette couche d'émulsion, qui sensibilise l'halogénure d'argent pour de la lumière dans le domaine spectral de 600 à 690 nm, caractérisé en ce que le colorant de sensibilisation répond à la formule générale (I) 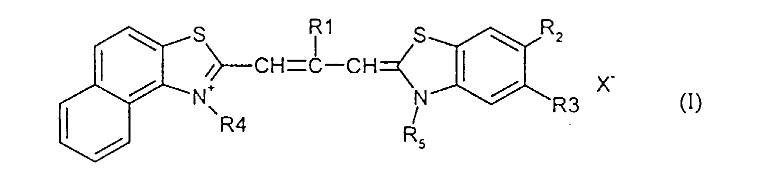 dans laquelle R₁ représente un groupe alkyle contenant de 1 à 6 atomes de carbone ou un noyau hétérocyclique, R₂ représente un groupe méthoxy, R₃ représente un atome d'hydrogène, un groupe méthyle ou un groupe méthoxy, R₄ et R₅ représentent des groupes alkyle contenant de 1 à 6 atomes de carbone, qui peuvent porter un ou plusieurs substituants hydroxyle, et X⁻ représente un anion.

2. Matériau selon la revendication 1, caractérisé en ce que la couche d'émulsion à l'halogénure d'argent est une couche à traitement négatif.

3. Matériau selon la revendication 1 ou 2, caractérisé en ce qu'il contient un composé d'hydrazine dans la couche d'émulsion à l'halogénure d'argent ou dans une couche se trouvant en liaison de réaction avec cette dernière.

4. Matériau selon l'une quelconque des revendications 1 à 3, caractérisé en ce qu'il contient un composé amino augmentant le contraste dans la couche d'émulsion à l'halogénure d'argent ou dans une couche se trouvant en liaison de réaction avec cette dernière.

5. Matériau selon l'une quelconque des revendications 1 à 4, caractérisé en ce que le colorant de sensibilisation appartient au groupe de colorants ci-après:

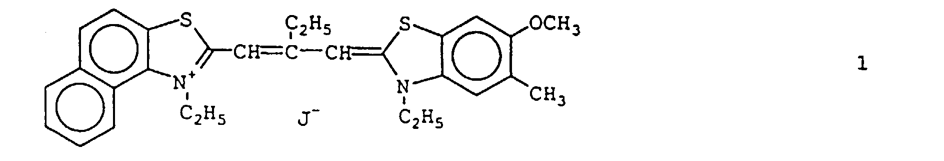

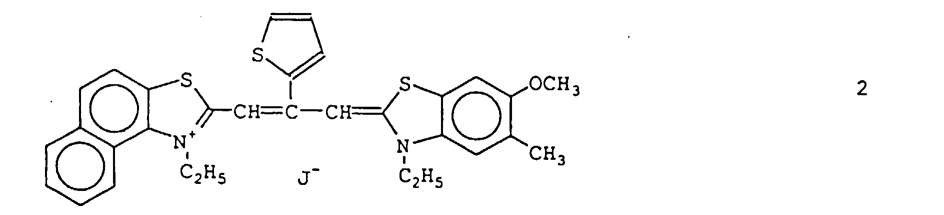

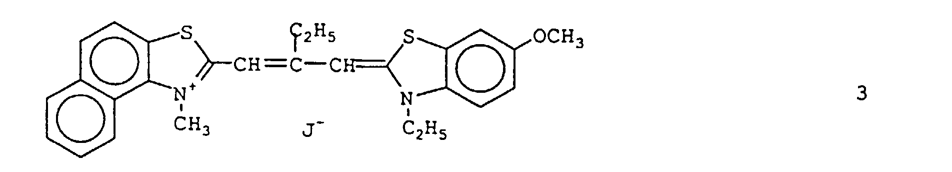

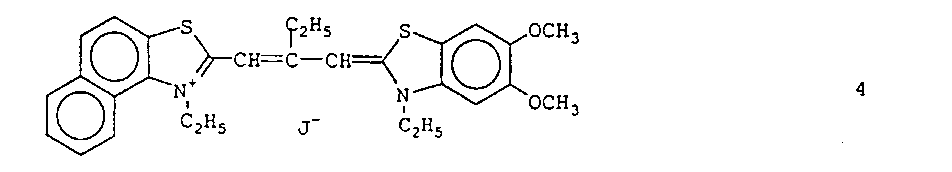

6. Matériau selon l'une quelconque des revendications 1 à 4, caractérisé en ce que l'émulsion à l'halogénure d'argent est une émulsion au bromure d'argent ou une émulsion au bromoiodure d'argent.

7. Procédé pour la préparation d'une image argentique, caractérisé en ce qu'on expose et on traite un matériau d'enregistrement photographique selon l'une quelconque des revendications précédentes.

8. Procédé selon la revendication 7, caractérisé en ce que le temps de développement s'élève au maximum à 30 secondes.
